(19) Europäisches Patentamt
European Patent Office
Office européen des brevets

(11) Publication number : **0 442 744 A2**

# (12) EUROPEAN PATENT APPLICATION

(21) Application number : **91301212.6**

(51) Int. Cl.[5] : **A61K 31/585,** A61K 31/705

(22) Date of filing : **14.02.91**

(30) Priority : **14.02.90 JP 33461/90**

(43) Date of publication of application :
**21.08.91 Bulletin 91/34**

(84) Designated Contracting States :
**AT BE CH DE DK ES FR GB GR IT LI LU NL SE**

(71) Applicant : **TSUMURA & CO.**
**4-10, 3-chome, Nihonbashi**
**Chuo-ku, Tokyo-to (JP)**
Applicant : **Tanaka, Yasuo**
**5-103-6 Midorigaoka**
**Yao-shi, Osaka-fu (JP)**

(72) Inventor : **Tanaka, Yasuo**
**5-103-6,Midorigaoka**
**Yao-shi, Osaka-fu (JP)**
Inventor : **Takechi,Masayuki**
**39-7, 3-chome, Tenmadainishi, Haibara-cho**
**Uda-gun, Nara-ken (JP)**

(74) Representative : **Myerscough, Philip Boyd et al**
**J.A. Kemp & Co. 14 South Square, Gray's Inn**
**London WC1R 5LX (GB)**

(54) **Treatment of viral disease by glycosides.**

(57) A use of a compound of the formula :

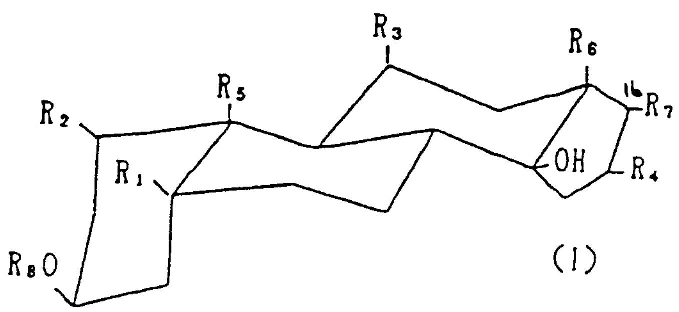

(I)

wherein
$R_1$, $R_2$, $R_3$ and $R_4$ are independently hydrogen atom or hydroxy group,
$R_5$ is hydrogen atom, methyl, hydroxymethyl, or formyl group,
$R_6$ is hydrogen atom or methyl group,
$R_7$ is 2,5-dihydro-2-oxofuran-4-yl group, or 2-oxo-2H-pyran-4-yl group, and
$R_8$ is hydrogen atom or sugar group,
with the proviso that, when $R_7$ is 2,5-dihydro-2-oxofuran-4-yl, then $R_8$ is not hydrogen atom,
or a 4,5-unsaturated compound corresponding thereto, as an active ingredient, for the manufacture of a medicament for the treatment of viral diseases.

EP 0 442 744 A2

# TREATMENT OF VIRAL DISEASE BY GLYCOSIDES

The present invention relates to a method for treatment of viral diseases by certain glycosides.

Rather few compounds have been noted as being excellent medicaments for use against viral diseases as compared with those against bacterial diseases. While not a few synthetic substances are known as being antivirally active, the most of them are not of practical use due to their insufficient activity or serious side-effects.

For example, some Herpes diseases such as Herpes simplex and Herpes zoster have long been known and also long been noted as being caused by viruses. In addition, within recent some 10 years, a new Herpes disease, a so-called fourth venereal disease, have spread with considerable rapidity. There have been only a few synthetic antiviral agents presented as being effective to these viral diseases but they have rarely if ever been practically used. Accordingly, there have been a continuous need for new antiviral agents having less or no side-effect.

It has been described that glycyrrhizin, a plant glycoside, is antivirally effective to infection of human immunodeficiency virus (HIV) (Antiviral Research, 7(1987), 127-137).

After an extensive study on the physiological activity of galenic and plant extracts, the inventors have discovered that some compounds within a group of steroid saponin, usually called "cardiac glycosides", and aglycons thereof have excellent antiviral activity.

In the first aspect, the present invention provides a method for treating viral diseases which comprises administering an antivirally effective amount of a compound of the formula :

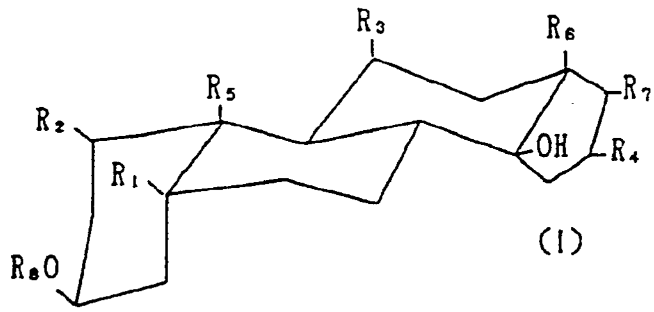

(I)

wherein

$R_1$, $R_2$, $R_3$ and $R_4$ are independently hydrogen atom or hydroxy group,
$R_5$ is hydrogen atom, methyl, hydroxymethyl, or formyl group,
$R_6$ is hydrogen atom or methyl group,
$R_7$ is 2,5-dihydro-2-oxofuran-4-yl group, or 2-oxo-2H-pyran-4-yl group, and
$R_8$ is hydrogen atom or sugar group,
with the proviso that, when $R_7$ is 2,5-dihydro-2-oxofuran-4-yl, then $R_8$ is not hydrogen atom,
or a 4,5-unsaturated compound corresponding thereto, as an active ingredient, to a subject in need of such treatment.

In the second aspect, the present invention provides a use of a compound of the formula (I) as defined above or a 4,5-unsaturated compound corresponding thereto, as an active ingredient, for the manufacture of a medicament for the treatment of viral diseases.

In the third aspect, the present invention provides an antiviral composition comprising, as an active ingredient, a compound of the formula (I) as defined above or a 4,5-unsaturated compound corresponding thereto, in association with a pharmaceutically acceptable carrier, diluent or excipient.

In the above formula, it is well known that the group 2,5-dihydro-2-oxolan-4-yl is in the relation of resonance with the group 2,3-dihydro-2-oxolan-4-yl and it is to be understood that these names refer to the same chemical group. In this specification, however, said chemical group is designated inclusively with the former expression only for the convenient sake.

The term "sugar group" includes residues of monosaccharides and oligosaccharides.

Within these residues, the residues of monosaccharides include residues of glucose, rhamnose, digitoxose, cymalose, thevelose, allomethylose, fucose, antiarose, digitalose, boivinose, sarmentose, acofriose, oleandrose etc. and the residues of oligosaccharides include residues, consisting of 2 to 6 monosaccharides, such as shown above, combined linearly or branchingly.

The compounds of the above formula (I) are characterized by the facts that their A/B rings have cis geometry, their B/C rings have trans geometry and their C/D rings have cis geometry. These compounds can

be obtained by extracting and purifying natural raw materials containing the compounds, such as animals or plants. Alternatively, these compound can be obtained synthetically by condensing an aglycon with a sugar in the presence of an appropriate enzyme according to the conventional enzymatic condensation process. Typical compounds and their source are described in Ullmann's Encyclopedia of Industrial Chemistry, fifth completely revised edition, Volume A5, pages 271-279 and T. Kariyone, "Plant Substance Chemistry" p.152-156 (in Japanese).

Examples of the typical compounds include digitoxin, gitoxin, digoxin, strophanthoside, strophanthin, cymarin, ouabain, convallatoxin, helveticoside, cheirotoxin, Thevetin A, Thevetin B, peruvoside, neriifolin, scillaren, proscillaridin, desglycohellebrin, bufalin, hellebringenin, scillarenin, scilliglaucosidin etc. These can be obtained be extracting plants or galenics of Scrophulariaceae, Apocynaceae, Asclepiadaceae, Cruciferae, Ranunculaceae, Celastraceae, Moraceae, Liliaceae etc., containing the compounds, separating and purifying the compounds by recrystallization, reprecipitation, chromatography etc.

As used herein, the expression "as an active ingredient" means that the compounds which can be used for the purpose of the invention includes, in addition to the compounds of the formula (I), relatively simple derivatives which release or reproduce the compounds of the formula (I) when said derivatives are introduced into the body, such as salts, complexes, esters etc. Examples of such derivatives include acetylated compounds at (a) hydroxy group(s) of the steroid nucleus or sugar residue, such as acetyldigitoxin, acetylgitoxin, acetyldigoxin, oleandrin etc.

As used herein, the term "antiviral" refers to a capacity of inhibiting or suppressing viral proliferation. Such capacity may be the basis for treatment of or treating viral diseases. The term "treatment" or "treating" means all management or control of disease including prevention, cure, remission, alleviation, prevention of aggravation etc.

The viral diseases to be treated according to the invention include human herpes infections such as herpes simplex, herpes zoster, caused by, for example, the Herpes simplex virus and Varicella zoster.

According to the invention, the active ingredient may be administered in the form of a pharmaceutical composition for oral administration or for injection but preferable for external application. Such composition includes ointments (such as oily ointment and hydrophilic ointment), lotion, liniment etc. The external composition may be prepared using, as the base or carrier, liquid paraffin, isopar, vaseline, silicone oil, aliphatic higher alcohols (cetyl alcohol, oleyl alcohol etc), higher fatty acids (myristic acid, stearic acid etc.), fatty acid esters (microcrystalline wax, isopropyl myristate etc.), lanolin, plastibase (a mixture of liquid paraffin and polyethylene), polyethylene glycol, water etc. Also, emulsifiers (fatty acid monoglyceride, sorbitan fatty acid ester, polyoxyethylene lauryl ether etc.), humectant (glycerol, propylene glycol, sorbitol, etc), antiseptic (methyl or propyl parahydroxybenzoate etc.), antioxidant (butyl hydroxyanisol etc.), pH-adjuster (citric acid etc.), suspending agent (carboxymethyl cellulose etc.) and other drugs (anti-itching agent, analgesic etc.) may be included. The external composition may be one for percutaneous absorption. Pharmaceutical compositions for oral administration (such as tablets and capsules) and those for injection can be prepared by the conventional process.

While the amount to be administered to the subject or to be contained in the composition may vary depending on the formulation, age of the subject, condition of the affected part etc., generally the concentration of 0.01 to 5 and preferably 0.1 to 0.5 microgram/gram base is suitable for the external composition. For oral administration or injection, the amount to be administered is about a half of the dose for usual cardiotonic administration. This is suitably 0.01 to 0.5 mg, for example 0.1 mg per a divided dose (three divided doses a day).

The present invention will be illustrated in more detail by the following Examples, in which the active ingredient may be any one of the compounds of formula (I).

Example 1

| | | |
|---|---|---|
| (A) | Active ingredient | 0.05 g |
| | Isopropyl myristate | 5 g |
| (B) | Plastibase | 94.95 g |

The ingredients in (A) are mixed and gradually added to (B) with stirring to form a homogeneous mixture: oily ointment.

Example 2

| | | |
|---|---|---|
| (A) | Active ingredient | 0.05 g |
| | Isopropyl myristate | 5.95 g |
| (B) | Isopropyl myristate | 10 g |
| | Vaseline | 66 g |
| | Liquid paraffin | 5 g |
| | Microcrystallin wax | 13 g |

The ingredients in (B) are melted by heating. To this are added the premixed ingredients in (A) at 45 to 50°C and the resulting mixture is stirred until it solidifies to form a homogeneous oily ointment.

Example 3

| | | |
|---|---|---|
| (A) | Active ingredient | 0.05 g |
| | Polyethylene glycol (400) | 11.95 g |
| (B) | Polyethylene glycol (400) | 12 g |
| | Polyethylene glycol (4,000) | 76 g |

The ingredients in (B) are melted at 70°C. To this are added the premixed ingredients in (A) at 50°C and the resulting mixture is stirred until it solidifies to form a homogeneous hydrophilic ointment. This ointment may further contain Carbopol 934 (Trademark).

Example 4

| | | |
|---|---|---|
| (A) | Active ingredient | 0.01 g |
| | Isopropyl myristate | 5.99 g |
| | Stearic acid | 19 g |
| | Cetyl alcohol | 4 g |
| (B) | Polyethylene lauryl ether | 2 g |
| | Glycerol monostearate | 0.5 g |
| | Propylene glycol | 4 g |
| | Citric acid | 0.05 g |
| (C) | Distilled water | 64.35 g |
| Methyl p-hydroxybenzoate | | 0.05 g |
| P-hydroxybenzoic acid | | 0.05 g |

The ingredients in (B) are melted at 80°C. To this are added the ingredients in (C) which are melted at 85°C and the ingredients in (A) at 55°C, and then the resulting mixture is stirred to form a homogeneous cream.

Example 5

| | | |
|---|---|---|
| (A) | Active ingredient | 0.001g |
| | Propylene glycol | 12 g |
| | Polyoxyethylene lauryl ether | 1 g |
| | Isostearic acid | 1 g |
| | Octyl dodecanol | 4 g |
| | Glycerol | 3 g |
| | Citric acid | 0.075g |
| (B) | Distilled water | 78.924g |

The ingredients in (A) are dissolved. To this is added the ingredient (B) with stirring to form a lotion.

Example 6

| | | |
|---|---|---|
| (A) | Active ingredient | 0.05 g |
| | Hydrocortisone acetate | 0.5 g |
| | Diphenhydramine | 0.5 g |
| | White vaseline | 4.85 g |
| (B) | White vaseline | 95 g |

The ingredients in (A) are melted by heating and mixed homogeneously with the ingredient (B) to form an ointment.

Example 7

Dispersed FL cells (established by J. Fogh and R. Lund from normal woman amnion. Dainippon Seiyaku Catalog No. 03-013) were cultivated in petri-dishes (diameter : 35mm) 24 hours before the start of the herpes virus inhibition experiment. When the experiment was started, the medium was removed from the dishes and a suspension (1 ml) containing Herpes simplex virus at the predetermined concentration in the medium was added to the dishes. The dishes were incubated in an incubator under the humidified air containing 5% $CO_2$ at 36°C for one hour to allow the adsorption of the viruses. Then the medium was removed and a fresh medium containing the test compounds was added. After 24 hours, the number of polykaryotic giant cells in each dish was counted and the minimum concentration showing no polykaryotic giant cells was taken as the antivirally active concentration to the herpes virus. The experiment was repeated without the virus and the minimum concentration at which cells died was taken as the cytotoxic concentration.

The following compounds were used as the test compounds.

| Compound No. | Trivial and Chemical Names, Origin |
|---|---|
| 1 | Digitoxin<br>3-[(O-2,6-dideoxy-β-D-ribo-hexopranoxyl-(1→4)-O-2,6-dideoxy-β-D-ribo-hexopyranosyl-(1→4)-2,6-dideoxy-β-D-ribo-hexopyranosyl)-oxy]-14-hydroxycard-(20)22-enolide<br>Extracted from Digitalis purpurea or commercially available |
| 2 | Digoxin<br>3-[(O-2,6-dideoxy-β-D-ribo-hexopranoxyl-(1→4)-O-2,6-dideoxy-β-D-ribo-hexopyranosyl-(1→4)-2,6-dideoxy-β-D-ribo-hexopyranosyl)-oxy]-12,14-dihydroxycard-(20)22-enolide<br>Extracted from Digitalis purpurea or commercially available |
| 3 | K-Strophanthin<br>a glycoside or a mixture of glycosides, said glycoside(s) being composed of an aglycon which is strophanthidin : 3,5,14-trihydroxy-19-oxocard-20(22)-enolide and sugars (for example, 2 x glucose plus cymalose)<br>Extracted from seeds of Strophanths gratus |

| | |
|---|---|
| 4 | Ouabain (G-Strophanthin)<br>3-[(6-deoxy-α-L-mannopyranosyl)oxy]-1,5,11α, 14,19-pentahydroxycard-20(22) enolide<br>Extracted from seeds of Strophanthus gratus or commercially available |
| 5 | Helveticoside<br>3β-[(2,6-dideoxy-β-D-ribo-hexopyranosyl)oxy-5,14-dihydroxy-19-oxo-5β-card-20(22) enolide<br>Extracted from Erysimum helveticum |
| 6 | Convallatoxin<br>3β-[(6-deoxy-α-L-mannopyranosyl)oxy]-5,14-dihydroxy-19-oxo-5β-card-20(22) enolide<br>Extracted from Convallaria majalis or Ornithogalum umbellatum, Synthetized from strophanthidin |
| 7 | Bufalin<br>3,14-dihydroxybufa-20,22-dienolide contained in poison of Bufo bufo gargorizans |

The results are shown in the following Table.

| Compound | Antivial activity(ng/ml) | Cytotoxity(ng/ml) |
|---|---|---|
| 1 | 25 | 480 |
| 2 | 50 | 480 |
| 3 | 50 | 570 |
| 4 | 40 | 460 |
| 5 | 30 | 460 |
| 6 | 25 | 380 |
| 7 | 8 | 500 |

It can be clearly seen from the above Table that the compounds tested have excellent antiviral activity and very low toxicity.

## Claims

1. Use of a compound of the formula :

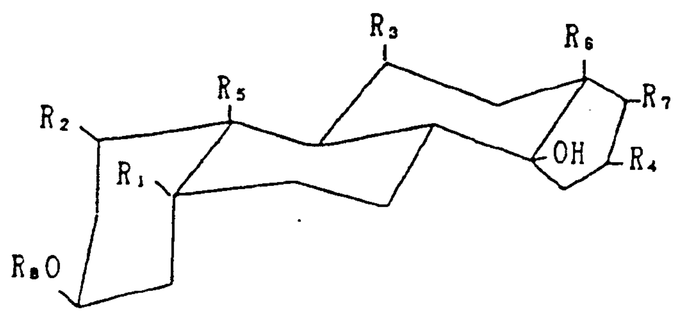

(I)

wherein
$R_1$, $R_2$, $R_3$ and $R_4$ are independently a hydrogen atom or hydroxy group,
$R_5$ is a hydrogen atom, methyl, hydroxymethyl or formyl group,
$R_6$ is a hydrogen atom or methyl group,
$R_7$ is 2,5-dihydro-2-oxofuran-4-yl group or 2-oxo-2H-pyran-4-yl group, and
$R_8$ is a hydrogen atom or sugar group,
with the proviso that when $R_7$ is 2,5-dihydro-2-oxofuran-4-yl then $R_8$ is not hydrogen atom,
or a 4,5-unsaturated compound corresponding thereto, as an active ingredient, for the manufacture of a medicament for use in the treatment of viral diseases.

2. Use according to claim 1, in which $R_5$ is hydrogen atom, hydroxymethyl or formyl group, and $R_8$ is rhaminosyl-, digitoxosyl-, cymalosyl-, digitoxosyl-digitoxosyl-digitoxosyl- or glucosyl-glucosyl-cymalosyl-.

3. Use according to claim 1 or 2, in which $R_7$ is 2,5-dihydro-2-oxofuran-4-yl.

4. Use according to claim 1 or 2, in which $R_7$ is 2-oxo-2H-pyran-4-yl group.

5. Use according to any one of the preceding claims, in which $R_1$, $R_2$, $R_3$, $R_4$, $R_5$, $R_6$ and $R_8$ are hydrogen atoms.

6. Use according to claim 1, in which the compound is oubain, helveticose, convallatoxin, cymarin, digitoxin, digoxin or strophanthin.

7. Use according to any one of the preceding claims, in which the disease is caused by a virus belonging to Herpesviridae.

8. Use according to claim 7, in which the disease is caused by a Herpes simplex virus.

9. Use according to claim 7, in which the disease is caused by a Varicella zoster virus.